(19) Europäisches Patentamt
European Patent Office
Office européen des brevets

(11) Veröffentlichungsnummer: **0 376 118 B1**

# (12) EUROPÄISCHE PATENTSCHRIFT

(45) Veröffentlichungstag der Patentschrift: **02.03.94**

(51) Int. Cl.⁵: **C08F 2/10**, A61L 15/24, A61L 15/60

(21) Anmeldenummer: **89123423.9**

(22) Anmeldetag: **19.12.89**

## (54) Verfahren zur Herstellung von feinteiligen, gelförmigen, wasserquellbaren Copolymerisaten.

(30) Priorität: **24.12.88 DE 3843780**

(43) Veröffentlichungstag der Anmeldung:
**04.07.90 Patentblatt 90/27**

(45) Bekanntmachung des Hinweises auf die Patenterteilung:
**02.03.94 Patentblatt 94/09**

(84) Benannte Vertragsstaaten:
**AT BE CH DE ES FR GB IT LI NL SE**

(56) Entgegenhaltungen:
**US-A- 4 286 082**

(73) Patentinhaber: **BASF Aktiengesellschaft**
**Carl-Bosch-Strasse 38**
**D-67063 Ludwigshafen(DE)**

(72) Erfinder: **Niessner, Manfred, Dr.**
**Budapester Strasse 51**
**D-6700 Ludwigshafen(DE)**
Erfinder: **Wickel, Stefan, Dr.**
**Giselherstrasse 71**
**D-6700 Ludwigshafen(DE)**
Erfinder: **Heide, Wilfried, Dr.**
**Am Wurmberg 16**
**D-6713 Freinsheim(DE)**
Erfinder: **Hartmann, Heinrich, Dr.**
**Weinheimer Strasse 46**
**D-6703 Limburgerhof(DE)**



Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Aus der US-PS 4 286 082 ist ein Verfahren zur Herstellung von wasserabsorbierenden Polymerisaten bekannt, bei dem man Alkalimetallacrylate oder Mischungen aus Alkalimetallacrylaten und Acrylsäure zusammen mit einem vernetzend wirkenden Monomer, das 2 bis 4 ethylenisch ungesättigte Doppelbindungen im Molekül enthält, in Gegenwart von oberflächenaktiven Stoffen in mindestens 25 gew.%iger wäßriger Lösung polymerisiert und das dabei entstehende gelförmige Polymer in der Wärme trocknet. Das oberflächenaktive Mittel hat dabei die Aufgabe, die Copolymerisation der wasserlöslichen Monomeren mit den Vernetzern zu verbessern. Es befindet sich nach Abschluß der Polymerisation auf der Oberfläche und im Innern der Gelteilchen. Das Tensid muß vor der Polymerisation zum Reaktionsgemisch zugegeben werden. Ein Zusatz des oberflächenaktiven Stoffs nach Abschluß der Polymerisation wirkt sich nach den Angaben in der Patentschrift negativ auf die Qualität des Produkts aus.

Aus der GB-A-2 146 343 ist ein Verfahren zur kontinuierlichen Herstellung von wasserabsorbierenden vernetzten Polymerisaten in einem zweiwelligen Kneter bekannt, bei dem man wäßrige Lösungen von gegebenenfalls partiell neutralisierten ethylenisch ungesättigten Carbonsäuren zusammen mit einem Vernetzer in Gegenwart von üblichen Polymerisationsinitiatoren copolymerisiert. Man erhält dabei Polymerteilchen, deren Durchmesser im allgemeinen 3 cm nicht überschreitet und üblicherweise in dem Bereich von 0,05 bis 1 cm liegt. Da die Partikelgröße einen direkten Einfluß auf die Diffusion des Wasser während des Trocknungsvorgangs hat, müssen größere Gelteilchen längere Zeit getrocknet werden als kleinere.

Aus der US-PS 4 769 427 ist ein Verfahren zur Herstellung von vernetzten feinteiligen, gelförmigen Polymerisaten in einwelligen Mischern mit hohem Selbstreinigungsgrad bekannt. Man unterwirft Monomermischungen, die pro 100 Gew.-Teile einer zu jeweils 50 bis 100 Mol% neutralisierten Acrylsäure oder Methacrylsäure, Acrylamid, Methacrylamid oder N-Vinylpyrrolidon sowie 0,01 bis 5 Gew.-Teile eines Vernetzers enthalten, in 20 bis 65 gew.%iger wäßriger Lösung in Gegenwart von Initiatoren der Polymerisation bei Temperaturen in dem Bereich von 45 bis 95 °C und entfernt während der Polymerisation unter vermindertem Druck einen Teil des Wassers aus der Reaktionsmischung, so daß ein krümmeliges Gel mit einem Feststoffgehalt von 30 bis 70 Gew.% ausgetragen wird.

Ein ähnliches Verfahren ist aus der EP-A-0 238 050 bekannt, bei dem die Copolymerisation z.B. von gegebenenfalls mit Alkalimetallbasen neutralisierter Acrylsäure oder Methacrylsäure und einem Vernetzer mehrstufig in einer diskontinuierlich arbeitenden Mischvorrichtung unter ständiger Durchmischung in allen Stufen durchgeführt wird, wobei man in der ersten Stufe der Polymerisation die wäßrige Monomerlösung bei Temperaturen von 45 bis 95 °C und einem Druck von 0,1 bis 0,8 bar unter partiellem Abdestillieren von Wasser copolymerisiert, in der zweiten Stufe die Copolymerisation bei Temperaturen von 100 bis 170 °C unter einem Druck bis zu 8 bar vervollständigt und nach dem Entspannen in der dritten Stufe dann den Wassergehalt des so hergestellten feinteiligen, vernetzten Copolymerisats auf Werte von 0,5 bis 10 Gew.% erniedrigt. Bei den beiden zuletzt genannten Verfahren wird ein krümmeliges Polymergel gebildet, das jeweils im Polymerisationsreaktor nur teilweise getrocknet werden kann. Während der Nachpolymerisation und während der Trocknung wird das Polymergel mehr oder weniger stark geschert, so daß es zu einer an sich ungewünschten Veränderung in der Gelstruktur kommt.

Der Erfindung liegt die Aufgabe zugrunde, ein Verfahren zur Herstellung von feinteiligen, gelförmigen, wasserquellbaren Copolymerisaten zur Verfügung zu stellen, bei dem möglichst feinteilige Gele anfallen, deren Struktur bei der Nachpolymerisation und Trocknung weitgehend erhalten bleibt, die nicht miteinander verkleben und die einfach gehandhabt und rasch getrocknet werden können.

Die Aufgabe wird erfindungsgemäß mit einem Verfahren zur Herstellung von feinteiligen, gelförmigen, wasserquellbaren Copolymerisaten durch Copolymerisieren von

(a) wasserlöslichen, monoethylenisch ungesättigten Monomeren,

(b) 0,001 bis 1 Mol-%, bezogen auf die Monomeren (a), mindestens zwei ethylenisch ungesättigte Doppelbindungen enthaltenden Monomeren und

(c) 0 bis 20 Mol-%, bezogen auf die Monomeren (a), wasserunlöslichen, monoethylenisch ungesättigten Monomeren

in 20 bis 80 gew.%iger wäßriger Lösung in Gegenwart von Polymerisationsinitiatorenund 0,1 bis 10 Gew.%, bezogen auf die bei der Copolymerisation eingesetzten Monomeren, an Tensiden, die einem HLB-Wert von mindestens 3 haben, bei Temperaturen oberhalb von 30 °C in Mischapparaturen mit einem Selbstreinigungsgrad oberhalb von 80% unter Durchmischen und anschließendem Trocknen der feinteiligen gelförmigen Copolymerisate gelöst, wenn man die die Monomeren in Gegenwart von zunächst 0 bis 50 Gew.% der Tensidmenge bis zu einem Umsatz von mindestens 60 % copolymerisiert, dann die restliche Tensidmenge zum Reaktionsgemisch zugibt und die Copolymerisation zu Ende führt.

Gibt man das Tensid wie oben angegeben zum Reaktionsgemisch, nachdem mindestens 60 % der Monomeren copolymerisiert sind, so zerfällt das Polymergel überraschenderweise innerhalb weniger Sekunden zu kleinen Partikeln. Diese feinteiligen Gele sind nicht klebrig, können leicht gerührt und unter vermindertem Druck im Reaktor getrocknet werden. Das Vorliegen besonders feinteiliger gelförmiger Copolymerisate nach der Tensidzugabe wird dadurch deutlich, daß man die Korngrößenverteilung der gelförmigen Copolymerisate nach einer Behandlung des Reaktionsgemisches mit Methanol mit Hilfe einer Siebanalyse bestimmen kann.

Wasserlösliche monoethylenisch ungesättigte Monomere der Gruppe (a) sind beispielsweise ethylenisch ungesättigte $C_3$- bis $C_6$-Carbonsäuren, deren Amide und Ester mit Aminoalkoholen der Formel

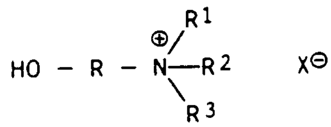

(I),

in der R = $C_2$- bis $C_5$-Alkylen, $R^1$, $R^2$, $R^3$ = H, $CH_3$, $C_2H_5$, $C_3H_7$ bedeutet. Bei diesen Verbindungen handelt es sich beispielsweise um Acrylsäure, Methacrylsäure, Crotonsäure, Itaconsäure, Maleinsäure, Fumarsäure, Acrylamid, Methacrylamid, Crotonsäureamid, Dimethylaminoethylacrylat, Diethylaminoethylacrylat,Dimethylaminopropylacrylat, Dimethylaminobutylacrylat, Diethylaminoethylmethacrylat, Dimethylaminoethylmethacrylat, Dimethylaminopropylacrylat, Dimethylaminoneopentylacrylat und Dimethylaminoneopentylmethacrylat. Die basischen Acrylate und Methacrylate werden in Form der Salze mit starken Mineralsäuren, Sulfonsäuren oder Carbonsäuren oder in quaternisierter Form eingesetzt. Das Anion $X^{\ominus}$ für die Verbindungen der Formel I ist der Säurerest der Mineralsäuren bzw. der Carbonsäuren oder Methosulfat, Ethosulfat oder Halogenid aus einem Quaternierungsmittel.

Weitere wasserlösliche Monomere der Gruppe (a) sind N-Vinylpyrrolidon, Acrylamidopropansulfonsäure, Vinylphosphonsäure und/oder Alkali- bzw. Ammoniumsalze der Vinylsulfonsäure. Die anderen Säuren können ebenfalls entweder in nicht neutralisierter Form oder in partiell bzw. bis zu 100 % neutralisierter Form bei der Polymerisation eingesetzt werden. Als wasserlösliche Monomere der Gruppe (a) eignen sich auch Diallylammoniumverbindungen, wie Dimethyldiallylammoniumchlorid, Diethyldiallylammoniumchlorid oder Diallylpiperidiniumbromid, N-Vinylimidazoliumverbindungen, wie Salze oder Quaternisierungsprodukte von N-Vinylimidazol und 1-Vinyl-2-methylimidazol, und N-Vinylimidazoline, wie N-Vinylimidazolin, 1-Vinyl-2-methylimidazolin, 1-Vinyl-2-ethylimidazolin oder 1-Vinyl-2-n-propylimidazolin, die ebenfalls in quaternisierter Form oder als Salz bei der Polymerisation eingesetzt werden. Bevorzugte Monomere der Gruppe (a) sind Acrylsäure, Methacrylsäure, Acrylamid und/oder Methacrylamid. Diese Monomeren können in jedem beliebigen Verhältnis miteinander copolymerisiert werden.

Die Polymerisation der Monomeren der Gruppe (a) erfolgt in Gegenwart von Vernetzern (Monomeren der Gruppe (b)). Die Vernetzer enthalten mindestens zwei ethylenisch ungesättigte Doppelbindunge., Geeignete Vernetzer sind beispielsweise N,N'-Methylen-bisacrylamid, Polyethylenglykoldiacrylate und Polyethylenglykoldimethacrylate, die sich jeweils von Polyethylenglykolen eines Molekulargewichts von 126 bis 8.500, vorzugsweise 400 bis 2000, ableiten, Trimethylolpropantriacrylat, Trimethylolpropantrimethacrylat, Ethylenglykoldiacrylat, Propylenglykoldiacrylat, Butandioldiacrylat, Hexandioldiacrylat, Hexandioldimethacrylat, Diacrylate und Dimethacrylate von Blockcopolymerisaten aus Ethylenoxid und Propylenoxid, zweifach bzw. dreifach mit Acrylsäure oder Methacrylsäure veresterte mehrwertige Alkohole, wie Glycerin oder Pentaerythrit, Triallylamin, Tetraallylethylendiamin, Divinylbenzol, Diallylphthalat, Polyethylenglykoldivinylether von Polyethylenglykolen eines Molekulargewichts von 126 bis 4000, Trimethylolpropandiallylether, Butandioldivinylether, Pentaerythrittriallylether und/oder Divinylethylenharnstoff. Vorzugsweise setzt man wasserlösliche Vernetzer ein, z.B. N,N'-Methylen-bisacrylamid, Polyethylenglykoldiacrylate, Polyethylenglykoldimethacrylate, Pentaerythrittriallylether und/oder Divinylharnstoff. Die Monomeren der Gruppe (b) werden in Mengen von 0,001 bis 1, vorzugsweise 0,005 bis 0,5 Mol-%, bezogen auf die Monomeren (a) bei der Copolymerisation eingesetzt.

Bei der Copolymerisation werden vorzugsweise als Monomer der Gruppe

(a) Acrylsäure, Methacrylsäure oder deren Gemische in Form der freien Säuren oder in partiell oder vollständig mit Alkalimetallbasen neutralisierter Form und als Monomer der Gruppe

(b) Acrylsäure- oder Methacrylsäure-diester von Polyethylenglykolen eines Molekulargewichts von 200 bis 2000,

eingesetzt.

Die Copolymerisation der Monomeren der Gruppen (a) und (b) kann - sofern eine Änderung der Eigenschaften der Copolymerisate gewünscht wird - zusätzlich noch in Gegenwart von Monomeren der Gruppe (c) durchgeführt werden. Als Monomere der Gruppe (c) kommen beispielsweise Hydroxyethylacrylat, Hydroxypropylacrylat, Hydroxyethylmethacrylat, Hydroxypropylmethacrylat, Acrylnitril und/oder Methacrylnitril in Betracht. Außerdem eignen sich Ester der Acrylsäure oder Methacrylsäure mit einwertigen, 1 bis 18 Kohlenstoffatome enthaltenden einwertigen Alkoholen, z.B. Methylacrylat, Ethylacrylat, Propylacrylat, Isopropylacrylat, n-Butylacrylat, Isobutylacrylat, Hexylacrylat, 2-Ethylhexylacrylat, Stearylacrylat, die entsprechenden Ester der Methacrylsäure, Fumarsäurediethylester, Maleinsäurediethylester, Maleinsäuredimethylester, Maleinsäuredibutylester, Vinylacetat und Vinylpropionat. Sofern die Monomeren der Gruppe (c) zur Modifizierung der wasserlöslichen Polymerisate verwendet werden, setzt man 0,5 bis 20, vorzugsweise 2 bis 10 Mol-%, bezogen auf die Monomeren (a) ein.

Die Polymerisation kann gegebenenfalls in Gegenwart der üblichen Polymerisationsregler erfolgen. Geeignete Polymerisationsregler sind beispielsweise Thioverbindungen, wie Thioglykolsäure, Mercaptoalkohole, z.B. 2-Mercaptoethanol, Mercaptopropanol und Mercaptobutanol, Dodecylmercaptan, Ameisensäure, Ammoniak und Amine, z.B. Ethanolamin, Diethanolamin, Triethanolamin, Triethylamin, Morpholin und Piperidin.

Die Monomeren (a), (b) und gegebenenfalls (c) werden in 20 bis 80, vorzugsweise 40 bis 60 gew.%iger wäßriger Lösung in Gegenwart von Polymerisationsinitiatoren miteinander copolymerisiert. Als Polymerisationsinitiatoren können sämtliche unter den Polymerisationsbedingungen in Radikale zerfallende Verbindungen eingesetzt werden, z.B. Peroxide, Hydroperoxide, Wasserstoffperoxid, Persulfate, Azoverbindungen und die sogenannten Redoxkatalysatoren. Bevorzugt ist der Einsatz von wasserlöslichen Katalysatoren. In manchen Fällen ist es vorteilhaft, Mischungen verschiedener Polymerisationsinitiatoren zu verwenden, z.B. Mischungen aus Wasserstoffperoxid und Natrium- oder Kaliumperoxidisulfat. Mischungen aus Wasserstoffperoxid und Natriumperoxidsulfat können in jedem beliebigen Verhältnis verwendet werden. Geeignete organische Peroxide sind beispielsweise Acetylacetonperoxid, Methylethylketonperoxid, tert.-Butylhydroperoxid, Cumolhydroperoxid, tert.-Amylperpivalat, tert.-Butylperpivalat, tert.-Butylperneohexanoat, tert.-Butylperisobutyrat, tert.-Butylper-2-ethylhexanoat, tert.-Butylperisononanoat, tert.-Butylpermaleat, tert.-Butylperbenzoat, tert.-Butylper-3,5,5-trimethylhexanoat und tert.-Amylperneodekanoat. Weitere geeignete Polymerisationsinitiatoren sind Azostarter, z.B. 2,2'-Azobis-(2-amidinopropan)dihydrochlorid, 2,2'-Azobis-(N,N'-dimethylen)isobutyramidin-dihydrochlorid, 2-(Carbamoylazo)isobutyronitril und 4,4'-Azobis-(4-cyanovaleriansäure). Die genannten Polymerisationsinitiatoren werden in üblichen Mengen eingesetzt, z.B. in Mengen von 0,01 bis 5, vorzugsweise 0,1 bis 2 Mol%, bezogen auf die zu polymerisierenden Monomeren.

Die Redoxkatalysatoren enthalten als oxidierende Komponente mindestens eine der oben angegebenen Perverbindungen und als reduzierende Komponente beispielsweise Ascorbinsäure, Glukose, Sorbose, Ammonium- oder Alkalimetall-hydrogensulfit, -sulfit, -thiosulfat, -hyposulfit, -pyrosulfit oder -sulfid, Metallsalze, wie Eisen-II-ionen oder Silberionen oder Natriumhydroxymethylsulfoxylat. Vorzugsweise verwendet man als reduzierende Komponente des Redoxkatalysators Ascorbinsäure oder Natriumpyrosulfit. Bezogen auf die bei der Polymerisation eingesetzte Menge an Monomeren verwendet man $3 \cdot 10^{-4}$ bis 1 Mol% der reduzierenden Komponente des Redoxkatalysatorsystems und 0,01 bis 5 Mol% der oxidierenden Komponente des Redoxkatalysators. Anstelle der oxidierenden Komponente des Redoxkatalysators kann man auch einen oder mehrere wasserlösliche Azostarter verwenden.

Zur Herstellung der feinteiligen, gelförmigen, wasserquellbaren Copolymerisate benötigt man Tenside. Geeignet sind hierfür alle Tenside, die einen HLB-Wert von mindestens 3 haben (zur Definition des HLB-Werts vgl. W.C. Griffin, J. Soc. Cosmetic Chem. Vol. 5, 249 (1954)). Geeignete nichtionische Tenside sind beispielsweise die Anlagerungsprodukte von Ethylenoxid, Propylenoxid oder Mischungen aus Ethylenoxid und Propylenoxid an Alkylphenole, aliphatische Alkohole, Carbonsäuren und Amine. Beispielsweise eignen sich mit Ethylenoxid und/oder Propylenoxid alkoxylierte $C_8$- bis $C_{12}$-Alkylphenole. Handelsübliche Produkte dieser Art sind beispielsweise Octylphenole bzw. Nonylphenole, die jeweils mit 4 bis 20 Mol Ethylenoxid pro Mol Phenol umgesetzt sind. Andere nichtionische Tenside sind ethoxylierte $C_{10}$- bis $C_{24}$-Fettalkohole oder ethoxylierte $C_{10}$- bis $C_{24}$-Fettsäuren sowie ethoxylierte $C_{10}$- bis $C_{24}$-Fettamine oder ethoxylierte $C_{10}$- bis $C_{24}$-Fettsäureamide. Außerdem eignen sich partiell mit $C_{10}$- bis $C_{24}$-Fettsäuren partiell veresterte mehrwertige $C_3$- bis $C_6$-Alkohole. Diese Ester können zusätzlich mit 2 bis 20 Mol Ethylenoxid umgesetzt sein. Als Fettalkohole, die zur Herstellung der Tenside alkoxyliert werden, eignen sich beispielsweise Palmitylalkohol, Stearylalkohol, Myristylalkohol, Laurylalkohol, Oxoalkohole sowie ungesättigte Alkohole, wie Oleylalkohol. Die Fettalkohole werden dabei zu einem solchen Grad ethoxyliert bzw. propoxyliert oder mit Ethylenoxid und Propylenoxid umgesetzt, daß die Reaktionsprodukte in Wasser löslich sind. Im allgemeinen setzt man 1 Mol der oben angegebenen Fettalkohole mit 2 bis 20 Mol Ethylenoxid und gegebenenfalls bis zu 5 Mol Propylenoxid so um, daß man Tenside erhält, die einen HLB-Wert von mehr als 8 haben.

$C_3$- bis $C_6$-Alkohole, die partiell verestert und gegebenenfalls ethoxyliert werden, sind beispielsweise Glycerin, Sorbit, Mannit und Pentaerythrit. Diese mehrwertigen Alkohole werden mit $C_{10}$- bis $C_{24}$-Fettsäuren z.B. Ölsäure, Stearinsäure oder Palmitinsäure partiell verestert. Die Veresterung mit den Fettsäuren erfolgt dabei höchstens bis zu einem solchen Grad, daß noch mindestens eine OH-Gruppe des mehrwertigen Alkohols unverestert bleibt. Geeignete Veresterungsprodukte sind beispielsweise Sorbitanmonooleat, Sorbitantristearat, Manitmonooleat, Glycerinmonooleat und Glycerindioleat. Die genannten Fettsäureester mehrwertiger Alkohole, die noch mindestens eine freie OH-Gruppe enthalten, können zur Modifizierung noch mit Ethylenoxid, Propylenoxid oder Mischungen aus Ethylenoxid und Propylenoxid umgesetzt werden. Pro Mol Fettsäureester verwendet man vorzugsweise 2 bis 20 Mol der genannten Alkylenoxide. Der Ethoxylierungsgrad hat bekanntlich einen Einfluß auf den HLB-Wert der nichtionischen Tenside. Durch geeignete Wahl der Alkoxylierungsmittel und der Menge an Alkoxylierungsmittel kann man Tenside mit HLB-Werten in dem Bereich von 3 bis 20 in technisch einfacher Weise herstellen.

Eine weitere Gruppe geeigneter Substanzen sind Homopolymere des Ethylenoxids, Blockcopolymere von Ethylenoxid und Alkylenoxiden, vorzugsweise Propylenoxid sowie polyfunktionelle Blockcopolymere, die beispielsweise durch sequentielle Addition von Propylenoxid und Ethylenoxid an Diamine gebildet werden.

Die nichtionischen Tenside können entweder allein oder auch in Mischung miteinander verwendet werden. Hierbei ergeben sich zahlreiche Variationsmöglichkeiten, z.B. kann man Tenside eines verschiedenen Ethoxylierungsgrades oder auch alkoxylierte Phenole zusammen mit ethoxylierten Fettalkoholen oder ethoxylierten Fettalkoholderivaten einsetzen. Weitere geeignete Tenside sind $C_8$- bis $C_{24}$-Alkylsulfonate, die vorzugsweise in Form der Alkalisalze eingesetzt werden, $C_8$- bis $C_{24}$-Alkylsulfate, die vorzugsweise in Form der Alkali- oder Trialkanolammoniumsalze eingesetzt werden, Sulfonbernsteinsäurediester, z.B das Natriumsalz von Sulfobernsteinsäure-di-(2-ethylhexyl)ester, Sulfobernsteinsäurehalbester, wie beispielsweise das Dinatriumsalz der Rizinolsäure-monoethanolamidosulfobernsteinsäure oder Dinatriumfettalkoholpolyglykolethersulfosuccinat, $C_8$- bis $C_{24}$-Alkylarylsulfonsäuren, z.B. das Dinatriumsalz von Dodecyldiphenyletherdisulfonsäure sowie die Schwefelsäurehalbester von Anlagerungsprodukten von Ethylenoxid an Alkylphenole oder Fettalkohole. Geeignete kationische Tenside sind beispielsweise die Anlagerungsprodukte von Alkylenoxiden an Fettamine bzw. Salze von Fettaminen, wie z.B. Pentaoxethylstearylammoniumacetat oder ethoxyliertes Methyloleinamin-Methosulfat sowie langkettige Alkylbenzyldimethylammoniumverbindungen, wie $C_{10}$- bis $C_{22}$-Alkyl-benzyl-dimethylammoniumchlorid. Weitere geeignete kationische Tenside sind die Salze von Fettaminen, z.B. Kokosfettammoniumacetat, quaternäre Fettsäureaminoester, z.B. Difettsäureisopropylesterdimethylammoniummethosulfat sowie quarternäre Fettsäureaminoamide, z.B. N-Undecylensäurepropylamido-N-trimethyl-ammoniummethosulfat. Ebenfalls geeignet sind amphothere Tenside, beispielsweise solche, im gleichen Molekül mindestens ein quarternäres Ammoniumkation und mindestens ein Carboxylat- oder Sulfonatanion tragen. Handelsübliche Produkte dieser Art sind beispielsweise Dimethylcarboxymethyl-Fettsäurealkylamidoammoniumbetaine oder 3-(3-Fettsäureamido-propyl)dimethylammonium-2-hydroxypropansulfonate. Die ionischen Tenside können allein oder auch in Mischung miteinander verwendet werden sofern die Tensidmischung in Wasser nicht zur Ausfällung der Tenside führt.

Die Tenside werden in Mengen von 0,1 bis 10, vorzugsweise 0,5 bis 5 Gew.%, bezogen auf die bei der Copolymerisation eingesetzten Monomeren, angewendet.

Die Copolymerisation erfolgt unter guter Durchmischung der Reaktionsteilnehmer. Da die Viskosität der Reaktionsmischung im Verlauf der Polymerisation stark ansteigt, benötigt man zur Polymerisation Mischapparaturen mit hohem Selbstreinigungsgrad. Der Selbstreinigungsgrad der in Betracht kommenden Mischvorrichtungen liegt oberhalb von 80 %. Geeignete Vorrichtungen, die einen hohen Selbstreinigungseffekt haben, sind beispielsweise in Chemie-Ingenieur-Technik Band 57, 1005 (1985) beschrieben. Bei diesen Vorrichtungen handelt es sich vorzugsweise um Kneter oder geeignet konstruierte Extruder. Die Mischvorrichtungen können dabei eine oder mehrere Wellen aufweisen. Die kontinuierliche Herstellung von vernetzten feinteiligen, gelförmigen Polymerisaten in einer solchen Vorrichtung mit hohem Selbstreinigungsgrad ist beispielsweise aus der US-PS 4 769 427 bekannt. Die Copolymerisation kann kontinuierlich oder diskontinuierlich durchgeführt werden.

Gemäß Erfindung wird das Tensid in spezieller Weise dem Reaktionsgemisch zugeführt. Man kann die Monomeren (a) bis (c) zunächst in Abwesenheit von Tensiden bis zu einem Umsatz von mindestens 60 %, vorzugsweise 80 % copolymerisieren und dann die gesamte Menge an Tensid zufügen oder man führt der Polymerisationsvorrichtung eine bereits Tensid enthaltende wäßrige Monomerlösung zu, wobei die Tensidmenge höchstens 50 Gew.% der insgesamt bei der Polymerisation eingesetzten Tensidmenge ausmacht. Die restliche Menge an Tensid wird erst dann dem Reaktionsgemisch zugegeben, wenn die Monomeren zu mindestens 60, vorzugsweise mindestens 80 %, umgesetzt sind. Beispielsweise kann man der bei der Copolymerisation eingesetzten wäßrigen Monomerlösung 0 bis 50 Gew.%, vorzugsweise 0 bis 20 Gew.%

der Tensidmenge zusetzen und dem Reaktionsgemisch dann bei einem Umsatz von mindestens 60, vorzugsweise 80 %, die restliche Tensidmenge, d.h. 50 bis 100, vorzugsweise 80 bis 100 Gew.% zugeben. Der Zusatz der Tenside zum Reaktionsgemisch nach mindestens 60 %igen Umsatz der Monomeren geschieht vorzugsweise in Form einer wäßrigen Lösung, die 5 bis 50 Gew.% eines oder mehrerer Tenside enthält. Die Tenside können jedoch gegebenenfalls auch in unverdünntem Zustand, z.B. in flüssiger Form, als Schmelze oder als Pulver, zu der polymerisierenden Reaktionsmischung zugegeben werden. Die Zugabe der Tensidlösung oder des Tensids nach mindestens 60 %igem Monomerumsatz erfolgt innerhalb einer relativ kurzen Zeitspanne, nämlich beispielsweise von 1 Sekunde bis 5 Minuten. Bei einer kontinuierlichen Polymerisation entsprechen die Zeitangaben der mittleren Verweilzeit in dem Bereich, wo das Tensid zudosiert wird. Sobald die gesamte Tensid-Menge zum Polymergel zugefügt wird, zerfällt es unter den Polymerisationsbedingungen innerhalb weniger Sekunden in kleine, nichtklebende Teilchen. Wie bereits erwähnt, tritt mit fortschreitender Polymerisation eine starke Viskositätserhöhung des Reaktionsgemisches ein. Bei einer diskontinuierlich geführten Polymerisation ist dieses Phänomen daran erkenntlich, daß die Leistungsaufnahme des Rührwerks stark ansteigt. Sobald man das Tensid nach mindestens 60 %igem Monomerumsatz zu dem gebildeten Polymergel zusetzt, registriert man einen starken Abfall der Leitungsaufnahme des Rührwerks. Die Leistungsaufnahme geht etwa auf einen Wert zurück, der vor Beginn der Polymerisation beim Rühren der beinahe wasserdünnen Polymerlösung gemessen wird. Aufgrund der Tatsache, daß das Gel nach Zusatz der restlichen Tensidmenge in feine Teilchen zerfällt, die nicht miteinander agglomerieren und auch nicht an den Wandungen und den Rührwellen der Polymerisationsapparatur haften, tritt bei dem erfindungsgemäßen Verfahren keine Schädigung des Polymergels beim Nachpolymerisieren und Trocknen ein. Die Polymerisation kann bei Normaldruck, unter vermindertem Druck oder auch unter erhöhtem Druck ablaufen. Es kann in einigen Fällen von Vorteil sein, schon während des Polymerisierens Wasser aus dem System zu entfernen. Mit Hilfe einer Siedekühlung ist es außerdem möglich, die Polymerisationstemperatur des Reaktionsgemisches konstant zu halten. Vorzugsweise wird die Reaktionsmischung während der Polymerisation nicht gekühlt, so daß die Temperatur beispielsweise von 40 °C auf bis zu 100 °C ansteigt. Nach dem Zusatz der restlichen Tensidmenge zum Reaktionsgemisch wird die Copolymerisation zu Ende geführt, d.h. die Monomeren sollen möglichst vollständig polymerisiert werden, z.B. zu 95 bis 99,5 %. In einigen Fällen lassen sich auch noch höhere Umsätze erzielen. Die feinteiligen, gelförmigen Copolymerisate werden dann getrocknet. Im Produktionsbetrieb erfolgt das Trocknen beispielsweise in Bandtrocknern, Wirbelschicht trocknern oder Schaufeltrocknern. Der geringe Teilchendurchmesser der gelförmigen Copolymerisate führt nicht nur zu einer Verkürzung der Trocknungszeit gegenüber den Trocknungszeiten bei den nach bisher bekannten Verfahren hergestellten vergleichbaren Copolymerisaten sondern auf diese Weise ist auch noch möglich, den Restmonomerengehalt der feinteiligen Gele zu erniedrigen.

Man erhält auf diese Weise feinteilige, gelförmige, wasserquellbare Copolymerisate, die aufgrund ihrer hohen Wasseraufnahme als wasserabsorbierendes Mittel in Hygieneartikeln, z.B. Windeln oder auch als Bodenverbesserungsmittel verwendet werden. Die Wasseraufnahme und Wasserretention der nach dem erfindungsgemäßen Verfahren erhältlichen Gele unterscheidet sich nicht von den Polymerisatgelen, die in Abwesenheit eines Tensids erhältlich sind oder zu deren Herstellung man bereits die gesamte Tensidmenge vor der Polymerisation eingesetzt hat.

Die Art der Tensidzugabe hat jedoch einen Einfluß auf die Menge an löslichen Anteilen in den Polymerisatgelen. Gibt man wie im Stand der Technik beschrieben, die gesamte Tensidmenge zur Monomerlösung, so enthalten die Polymergele deutlich höhere lösliche Anteile wie die Polymergele, die nach dem erfindungsgemäßen Verfahren herstellbar sind und bei dem die Monomerlösung kein oder bis zu 50 Gew.% der erforderlichen Tensidmenge enthält und bei dem das gesamte Tensid oder die restliche Tensidmenge nach einem Monomerumsatz von mindestens 60 Gew.% zugesetzt wird. Ein niedriger Gehalt an löslichen Anteilen im Polymergel ist von anwendungstechnischem Vorteil.

Die Prozentangaben in den Beispielen sind Gewichtsprozent. Folgende Tenside wurden verwendet:

Tensid 1: Natriumsalz einer $C_{14}$-Alkylsulfonsäure

Tensid 2: Natriumsalz des Sulfonbernsteinsäure-di-(2-ethylhexyl)esters

Tensid 3: Dinatriumsalz von Dodecyldiphenylether-disulfonsäure

Tensid 4: Dinatriumsalz von Rizinolsäuremonoethanolamidosulfosuccinat

Tensid 5: Natriumlaurylsulfat

Tensid 6: Natriumsalz des Schwefelsäurehalbesters eines Umsetzungsprodukts von 25 Mol Ethylenoxid mit 1 Mol Isooctylphenol

Tensid 7: Anlagerungsprodukt von 8 Mol Ethylenoxid an 1 Mol $C_9$-Alkylphenol

Tensid 8: Anlagerungsprodukt von 5 Mol Ethylenoxid an 1 Mol eines $C_{13}$-/$C_{15}$-Oxoalkohols

Tensid 9: Anlagerungsprodukt von 50 Mol Ethylenoxid an 1 Mol eines $C_{16}$-/$C_{18}$-Fettalkohols

Tensid 10: Anlagerungsprodukt von 12 Mol Ethylenoxid an 1 Mol Rizinusöl
Tensid 11: Anlagerungsprodukt von 7 Mol Ethylenoxid an 1 Mol Oleinamin-methosulfat
Tensid 12: Dimethylcarboxymethyl-laurinsäurepropylamidoammoniumbetain
Tensid 13: $C_{12}$-/$C_{14}$-Alkyl-benzyl-dimethylammoniumchlorid
Tensid 14: Polyethylenglykol des Molekulargewichts 300
Tensid 15: Sorbitanmonooleat

Beispiele 1 bis 15 - Allgemeine Herstellvorschrift

1000 g einer 43 %igen wäßrigen Lösung aus 75 Mol-% Natriumacrylat und 25 Mol-% Acrylsäure werden mit 26 ml einer 0,5 %igen wäßrigen Lösung von N,N-Methylenbisacrylamid sowie mit 3 g einer 1 %igen wäßrigen Lösung von Diethylentriaminpentanatriumacetat versetzt. Diese Monomerlösung wird in einen Kneter gefüllt, dessen Wandtemperatur 60 °C beträgt. Sobald die Temperatur der Monomerlösung 40 °C erreicht, fügt man unter ständigem Durchmischen zunächst 4,3 g Natriumperoxidisulfat gelöst in 25 ml Wasser und danach eine Lösung von 0,13 g Natriumdisulfit in 25 ml Wasser zu. Der Beginn der Polymerisation ist an der Temperaturerhöhung des Reaktionsgemisches erkennbar. In Tabelle 1 sind die Temperaturen angegeben, auf die sich das Reaktionsgemisch maximal erwärmte sowie die jeweils erreichten Monomerumsätze, bei denen dann 1 %, bezogen auf die bei der Polymerisation ursprünglich eingesetzten Monomeren, eines Tensids zudosiert wurde, das ebenfalls in Tabelle 1 angegeben ist. Die Tensidzugabe zum Reaktionsgemisch führte innerhalb weniger Sekunden zu einem Zerfall des ursprünglich zusammenhängenden Polymergels in kleine Partikeln. Gleichzeitig ging die während der Polymerisationsreaktion angestiegene Leistungsaufnahme des Rührwerks auf einen Wert zurück, der vor Beginn der Polymerisations beim Rühren der beinahe wasserdünnen Monomerlösung gemessen wurde. Etwa 3 bis 5 Minuten nach Zugabe der Tensidlösung wurde der Druck im Reaktor sukzessive reduziert, so daß ein Teil des vorhandenen Wassers abdestillierte. Unter diesen Bedingungen wurde die Polymerisation zu Ende geführt, d.h. bis zu einem Monomer-Umsatz von ca. 99 %.

Bei allen Beispielen wurden jeweils 100 g des so hergestellten Polymergels in 1000 ml Methanol eingebracht und darin 30 Minuten mit einem Propellerrührer bei 300 Umdrehungen/Minute gerührt. Das wasserhaltige, methanolfeuchte Gel wurde abgesaugt und einer Siebanalyse unterworfen. Das restliche unbehandelte Gel wurde im Vakuumtrockenschrank getrocknet, gemahlen und gesiebt. An dem trockenen Pulver wird die Absorptionsfähigkeit von Wasser bestimmt. In Tabelle 1 sind die bei den Beispielen jeweils verwendeten Tenside, das Ergebnis der Siebanalysen, die Menge an abdestilliertem Nasser während der Nachpolymerisation sowie die Absorptionskapazität der feinteiligen, gelförmigen wasserquellbaren Copolymerisate angegeben.

Bestimmung der Absorptionskapazität

Die Absorptionskapazität wird mit Hilfe des sogenannten "Teebeuteltests" durchgeführt. Als Flüssigkeit dient dabei eine 0,9 %ige Kochsalzlösung. Eine definierte Menge an gelförmigem, wasserabsorbierenden Copolymerisat (1 g) wird in einen Teebeutel gefüllt, der dann verschlossen wird. Die Abmessungen des Teebeutels müssen der Menge des eingewogenen gelförmigen Copolymerisats entsprechend angepaßt sein. Der Teebeutel wird dann eine bestimmte Zeit lang in die Flüssigkeit eingetaucht und dann nach einer Abtropfdauer von 15 Sekunden zurückgewogen. Für die Berechnung der Absorptionskapazität muß ein Blindversuch durchgeführt werden, bei dem ein Teebeutel ohne gelförmiges, wasserabsorbierendes Copolymerisat in die Lösung eingetaucht wird und das Gewicht des Teebeutels nach der oben angegebenen Abtropfdauer bestimmt wird. Die Absorptionskapazität ergibt sich dann aus folgender Beziehung

$$\text{Absorptionskapazität} = \frac{\text{Gewicht des Teebeutels mit Polymerisatgel} - \text{Gewicht des Teebeutels im Blindversuch}}{\text{Gewicht des eingewogenen Polymerisatgels}}$$

Vergleichsbeispiel 1

Die oben angegebenen Beispiele wurden mit der Ausnahme wiederholt, daß man die Copolymerisation jetzt jeweils in Abwesenheit eines Tensids durchführte. Hierbei erhielt man ein grob krümmeliges Gel, dessen Partikelgröße auch noch nach Erreichen der maximalen Temperatur stetig zunahm. Die agglomerierten gelförmigen Teilchen wurden stark geknetet, kompaktiert und geschert. Dieser Befund war an der ständig ansteigenden Leistungsaufnahme des Rührers zu erkennen. Sobald der Druck im Reaktor abgesenkt wurde und Wasser abdestillierte, erhöhte sich die Leistungsaufnahme des Rührers so stark, daß die versuche bereits nach dem Abdestillieren von einigen ml Wasser abgebrochen werden mußten. Das Polymerisat lag in Form einer teigartigen, zähen, zusammenhängenden Masse vor. Der Austrag einzelner Partikeln und die Anfertigung einer Siebanalyse war nicht möglich.

Vergleichsbeispiel 2

Das Beispiel 4 wird mit der Ausnahme wiederholt, daß das darin verwendete Tensid - ebenfalls in einer Menge von 1 % - bezogen auf die bei der Polymerisation eingesetzten Monomeren, bereits vor Beginn der Polymerisation zu der wäßrigen Monomerlösung zugesetzt wurde. In diesem Fall erhielt man ein grob krümmeliges klebriges Polymergel, dessen Polymerteilchen beim Abdestillieren von Wasser stark agglomerierten, wodurch die Leistungsaufnahme des Rührers stark anstieg. Nach dem Abdestillieren von 30 ml Wasser mußte der Versuch abgebrochen werden, eine Siebanalyse des erhaltenen grobkörnigen Produkts sowie die Absorptionskapazität des Copolymerisats sind in Tabelle 1 angegeben.

Tabelle 1

| Bsp.-Nr. | T[1)] max. [°C] | Tensid | Tensidzugabe nach Umsatz von ... % | Siebanalyse >4 mm [%] | >3 mm [%] | >2 mm [%] | >1 mm [%] | <1 mm [%] | Summe <2 mm [%] | abdestilliertes Wasser [ml] | Absorptionskapazität der Polymergele [g/g] |
|---|---|---|---|---|---|---|---|---|---|---|---|
| 1 | 69 | Tensid 1 | 89 | 35,8 | 15,9 | 23,4 | 20,0 | 4,8 | 24,8 | 50 | 40 |
| 2 | 68 | Tensid 2 | 92 | 25,6 | 13,9 | 25,8 | 28,7 | 6,0 | 34,7 | 250 | 40 |
| 3 | 68 | Tensid 3 | 75 | 25,2 | 12,4 | 23,9 | 28,3 | 10,2 | 38,5 | 200 | 45 |
| 4 | 68 | Tensid 4 | 96 | 16,0 | 13,2 | 22,5 | 35,4 | 12,9 | 48,3 | 150 | 40 |
| 5 | 68 | Tensid 5 | 84 | 26,6 | 12,8 | 28,0 | 30,4 | 2,1 | 32,7 | 80 | 44 |
| 6 | 68 | Tensid 6 | 80 | 24,2 | 15,1 | 30,1 | 25,5 | 5,2 | 30,7 | 50 | 40 |
| 7 | 70 | Tensid 7 | 95 | 44,0 | 16,4 | 20,6 | 15,2 | 3,8 | 19,0 | 0 | 45 |
| 8 | 68 | Tensid 8 | 87 | 30,6 | 15,0 | 23,5 | 25,3 | 5,7 | 31,0 | 50 | 40 |
| 9 | 68 | Tensid 9 | 85 | 14,3 | 11,2 | 31,5 | 36,1 | 6,8 | 42,9 | 60 | 50 |
| 10 | 69 | Tensid 10 | 90 | 21,1 | 15,1 | 31,6 | 28,4 | 3,9 | 32,3 | 60 | 38 |
| 11 | 73 | Tensid 11 | 84 | 16,0 | 13,0 | 25,1 | 34,1 | 11,9 | 46,0 | 200 | 41 |
| 12 | 69 | Tensid 12 | 93 | 18,0 | 11,4 | 23,9 | 36,4 | 11,3 | 47,7 | 200 | 39 |
| 13 | 69 | Tensid 13 | 85 | 23,6 | 13,0 | 26,6 | 30,0 | 6,7 | 36,7 | 40 | 48 |
| 14 | 68 | Tensid 14 | 96 | 47,5 | 11,4 | 19,2 | 17,9 | 3,9 | 21,8 | 20 | 48 |
| 15 | 76 | Tensid 15 | 93 | 19,5 | 13,7 | 30,3 | 31,0 | 5,5 | 36,5 | 200 | 37 |
| Vergleichsbeispiel | | | | | | | | | | | |
| 2 | 69 | Tensid 4 | vor Beginn der Polymerisation | 59,4 | 11,2 | 14,0 | 12,0 | 3,3 | 15,3 | 30 | 42 |

1) bei der Polymerisation maximal erreichte Temperatur

## Beispiele 16 bis 21

1000 g einer 43 %igen wäßrigen Lösung aus 61 Mol-% Natriumacrylat und 39 Mol-% Acrylsäure werden mit 1,7 g eines Polyethylenglykoldiacrylats, dessen Polyethylenglykolsegment ein Molekulargewicht

von 300 hat, sowie mit 3 g einer 1 %igen wäßrigen Lösung von Diethylentriaminpentanatriumacetat versetzt. Die wäßrige Monomerlösung wird in einem vorgeheizten Kneter gefüllt, der eine Wandtemperatur von 60 °C hat. Sobald die Temperatur der wäßrigen Monomerlösung im Kneter 40 °C erreicht, gibt man zunächst 1 g Kaliumperoxidisulfat, gelöst in 25 ml Wasser, und danach eine Lösung von 0,08 g Natriumdisulfit in 25 ml Wasser zu. Der Polymerisationsbeginn ist an einer Temperaturerhöhung erkennbar. Die Temperatur steigt auf die in Tabelle 2 angegebenen Maximalwerte ($T_{max}$) an. 1 Minute nach Erreichen der maximalen Reaktionstemperatur liegen die in Tabelle 2 angegebenen Umsätze vor. Dann dosiert man innerhalb von 5 Sekunden 1 % des in Tabelle 2 angegebenen Tensids jeweils zu. Das ursprünglich grob krümmelige Gel wird nach der Tensidzugabe innerhalb weniger Sekunden in kleine Partikeln zerteilt. Gleichzeitig geht die während der Polymerisationsreaktion angestiegene Leistungsaufnahme des Rührwerks auf etwa den Wert zurück, der vor Beginn der Polymerisations beim Rühren der beinahe wasserdünnen Monomerlösung gemessen wurde. Etwa 3 bis 5 Minuten nach Zugabe des Tensids wird der Druck im Reaktor sukzessive auf 100 mbar reduziert, so daß ein Teil des vorhandenen Wassers abdestilliert und die Polymerisation zu Ende geführt. Die Menge des Destillats ist in Tabelle 2 angegeben. Der Umsatz der Monomeren beträgt danach 99 %. Wie in den Beispielen 1 bis 15 beschrieben, werden anschließend die Partikelgrößenverteilungen der Polymerisatgele bestimmt. Die Ergebnisse sind in Tabelle 2 zusammengefaßt.

Vergleichsbeispiel 3

Die Beispiele 16 bis 21 werden mit der Ausnahme wiederholt, daß man die Copolymerisation in Abwesenheit eines Tensids vornimmt. Das dabei erhaltene Polymergel agglomeriert während der Nachreaktion zu ständig wachsenden Partikeln, die nach Absenkung des Reaktorinnendrucks rasch zu einer zusammenhängenden Masse verkleben. Dabei wird das Polymergel ständig stark geschert, geknetet und kompaktiert. Die rasch ansteigende Leistungsaufnahme des Rührers führt schließlich zum Rührerstillstand. Die zusammenhängende Masse ließ sich erst nach mechanischem Zerteilen aus dem Kneter austragen, eine Siebanalyse war daher nicht möglich.

Vergleichsbeispiel 4

Das Beispiel 19 wurde in der Weise wiederholt, daß man die gesamte Menge des Tensids 9 - ebenfalls 1 %, bezogen auf die bei der Copolymerisation eingesetzten Monomeren - bereits der wäßrigen Monomerlösung zusetzte. Die Leistungsaufnahme des Rührers nahm auch nach Erreichen der Maximaltemperatur ständig zu und besonders rasch nach Absenkung des Reaktorinnendrucks. Der Versuch wurde nach dem Abdestillieren von 30 ml Wasser abgebrochen. Die Teilchengrößenverteilung der so erhältlichen Gelpartikeln ist in Tabelle 2 angegeben.

Tabelle 2

| Bsp.-Nr. | $T_{max}$ [°C] | Tensid | Tensidzugabe nach Umsatz von ... % | Siebanalyse >4 mm [%] | >3 mm [%] | >2 mm [%] | >1 mm [%] | <1 mm [%] | Summe <2 mm [%] | abdestilliertes Wasser [ml] |
|---|---|---|---|---|---|---|---|---|---|---|
| 16 | 87 | Tensid 2 | 96 | 9,7 | 7,7 | 17,2 | 42,6 | 22,8 | 65,4 | 100 |
| 17 | 90 | Tensid 3 | 75 | 2,4 | 4,0 | 17,2 | 58,6 | 17,9 | 76,5 | 250 |
| 18 | 93 | Tensid 4 | 87 | 1,7 | 1,7 | 9,9 | 48,7 | 38,1 | 86,8 | 40 |
| 19 | 87 | Tensid 9 | 89 | 2,0 | 3,0 | 12,7 | 57,0 | 25,4 | 82,4 | 50 |
| 20 | 90 | Tensid 11 | 93 | 8,2 | 6,6 | 18,0 | 45,7 | 21,6 | 67,3 | 50 |
| 21 | 95 | Tensid 12 | 93 | 1,0 | 1,6 | 12,1 | 58,6 | 26,8 | 85,4 | 100 |
| Vergleichsbeispiel | | | | | | | | | | |
| 4 | 95 | Tensid 9 | - | 41,2 | 3,9 | 10,8 | 25,8 | 18,3 | 44,1 | 30 |

Beispiel 22

Beispiel 4 wurde mit Vernetzerkonzentrationen von
a) 0,03 Gew.% und

b) 0,10 Gew.%

wiederholt. Man bestimmte dann jeweils die löslichen Anteile des Polymers. Folgende Werte wurden ermittelt:

a) 10 Gew.% und

b) 2,9 Gew.%.

Vergleichsbeispiel 5

Das Vergleichsbeispiel 2 wurde mit Vernetzerkonzentrationen von

a) 0,03 Gew.% und

b) 0,10 Gew.%

wiederholt. Man bestimmte dann jeweils die im Polymergel enthaltenen löslichen Anteile, die bei

a) 13,7 Gew.% und bei

b) 6,1 Gew.%

betrugen.

Ein Vergleich mit den Ergebnissen von Beispiel 22 zeigt, daß die nach dem Verfahren der US-PS 4 286 082 hergestellten Copolymerisate signifikant höhere lösliche Anteile enthalten als Copolymerisatgele gemäß Erfindung.

**Patentansprüche**

**1.** Verfahren zur Herstellung von feinteiligen, gelförmigen wasserquellbaren Copolymerisaten durch Copolymerisieren von

(a) wasserlöslichen, monoethylenisch ungesättigten Monomeren,

(b) 0,001 bis 1 Mol-%, bezogen auf die Monomeren (a), mindestens zwei ethylenisch ungesättigte Doppelbindungen enthaltenden Monomeren und

(c) 0 bis 20 Mol-%, bezogen auf die Monomeren (a), wasserunlöslichen, monoethylenisch ungesättigten Monomeren

in 20 bis 80 gew.%iger wäßriger Lösung in Gegenwart von Polymerisationsinitiatorenund 0,1 bis 10 Gew.%, bezogen auf die bei der Copolymerisation eingesetzten Monomeren, an Tensiden, die einen HLB-Wert von mindesten 3 haben bei Temperaturen oberhalb von 30 °C in Mischapparaturen mit einem Selbstreinigungsgrad oberhalb von 80% unter Durchmischen und anschließendem Trocknen der feinteiligen gelförmigen Copolymerisate, dadurch gekennzeichnet, daß man die Monomeren in Gegenwart von zunächst 0 bis 50 Gew.% der Tensidmenge bis zu einem Umsatz von mindestens 60 % copolymerisiert, dann die restliche Tensidmenge zum Reaktionsgemisch zugibt und die Copolymerisation zu Ende führt.

**2.** Verfahren nach Anspruch 1, dadurch gekennzeichnet, daß man die Monomeren in Gegenwart von zunächst 0 bis 50 Gew.% der Tensidmenge bis zu einem Umsatz von mindestens 80 % copolymerisiert.

**3.** Verfahren nach Anspruch 2, dadurch gekennzeichnet, daß man die Monomeren der Gruppe (a) mit wasserlöslichen Monomeren der Gruppe (b) zunächst in Abwesenheit von Tensiden bis zu einem Umsatz von mindestens 80 % copolymerisiert und dann die gesamte Tensidmenge zusetzt.

**4.** Verfahren nach einem der Ansprüche 1 bis 3, dadurch gekennzeichnet, daß man als Monomer der Gruppe

(a) Acrylsäure, Methacrylsäure oder deren Gemische in Form der freien Säuren oder in partiell oder vollständig mit Alkalimetallbasen neutralisierter Form und als Monomer der Gruppe

(b) Acrylsäure- oder Methacrylsäure-diester von Polyethylenglykolen eines Molekulargewichts von 200 bis 2000,

einsetzt.

**Claims**

**1.** A process for the preparation of a finely divided, gel-like, water-swellable copolymer, by copolymerizing

(a) water-soluble, monoethylenically unsaturated monomers,

(b) from 0.001 to 1 mol %, based on the monomers (a), of monomers containing two or more ethylenically unsaturated double bonds and

(c) from 0 to 20 mol %, based on the monomers (a), of water-insoluble, monoethylenically unsaturated monomers

in 20-80% strength by weight aqueous solution in the presence of a polymerization initiator and from 0.1 to 10% by weight, based on the monomers used in the copolymerization, of a surfactant which has an HLB value of not less than 3, at above 30 °C in a mixing apparatus having a degree of self-purging greater than 80 %, with thorough mixing and subsequent drying of the finely divided, gel-like copolymer, wherein the monomers are copolymerized in the presence of initially from 0 to 50 % by weight of the amount of surfactant to a conversion of not less than 60 %, then the remaining amount of surfactant is added to the reaction mixture and the copolymerization completed.

**2.** A process as claimed in claim 1, wherein the monomers are copolymerized in the presence of initially from 0 to 50% by weight of the amount of surfactant to a conversion of not less than 80%.

**3.** A process as claimed in claim 2, wherein the monomers of group (a) are copolymerized with water-soluble monomers of group (b), initially in the absence of surfactants, to a conversion of not less than 80%, and the total amount of surfactant is then added.

**4.** A process as claimed in any of claims 1 to 3, wherein as monomers of group

(a) acrylic acid, methacrylic acid or mixtures thereof in the form of the free acids or in a form partially or completely neutralized with alkali metal bases are used, and, as monomers of group

(b) acrylic or methacrylic diesters of polyethylene glycols having a molecular weight of from 200 to 2,000 are used.

## Revendications

**1.** Procédé de fabrication de copolymères finement divisés, géliformes, gonflables à l'eau, par la copolymérisation

(a) de monomères monoéthyléniquement insaturés, solubles dans l'eau,

(b) de 0,001 à 1% molaire, par rapport aux monomères (a), de monomères contenant au moins deux doubles liaisons éthyléniquement insaturées et

(c) de 0 à 20% molaires, par rapport aux monomères (a), de monomères monoéthyléniquement insaturés, insolubles dans l'eau,

en solution aqueuse à 20-80% en poids, en présence d'amorceurs de polymérisation et de 0,1 à 10% en poids, par rapport aux monomères mis en oeuvre au cours de la polymérisation, de composés tensioactifs ou surfactifs, qui possèdent une valeur HLB d'au moins 3, à des températures supérieures à 30 °C, dans des appareils mélangeurs avec un degré d'autoépuration supérieur à 80%, sous mélange intime et séchage subséquent des copolymères géliformes finement divisés, caractérisé en ce que l'on copolymérise les monomères d'abord en présence de 0 à 50% en poids de la proportion de substances tensioactives ou surfactives, jusqu'à une conversion d'au moins 60%, puis on ajoute la proportion résiduelle de substances tensioactives ou surfactives au mélange réactionnel et on poursuit la copolymérisation jusqu'à son achèvement.

**2.** Procédé suivant la revendication 1, caractérisé en ce que l'on copolymérise les monomères d'abord en présence de 0 à 50% en poids de la proportion des substances tensioactives ou surfactives jusqu'à une conversion d'au moins 80%.

**3.** Procédé suivant la revendication 2, caractérisé en ce que l'on copolymérise les monomères du groupe (a) avec des monomères solubles dans l'eau du groupe (b) d'abord en l'absence de substances tensioactives ou surfactives jusqu'à une conversion d'au moins 80% et on ajoute ensuite la proportion totale de substances tensioactives ou surfactives.

**4.** Procédé suivant l'une quelconque des revendications 1 à 3, caractérisé en ce que l'on utilise, à titre de monomère du groupe (a),

l'acide acrylique, l'acide méthacrylique ou leurs mélanges, sous forme des acides lbires ou sous forme partiellement ou totalement neutralisée avec des bases à métaux alcalins et, à titre de monomères du groupe (b),

des diesters de l'acide acrylique ou de l'acide méthacrylique de polyéthylèneglycols d'un poids moléculaire de 200 à 2000.